# EUROPEAN PATENT APPLICATION

(11) **EP 3 517 141 A1**
(43) Date of publication of application: **31.07.2019**
(21) Application number: 19153215.9
(22) Date of filing: 23.01.2019
(51) Int. Cl.: A61L 15/26, A61L 15/44, A61L 15/64

(54) **DRESSING FOR ACUTE OR CHRONIC WOUND**

(30) Priority: 26.01.2018 CZ 201834641 U
(71) Applicant: Technicka univerzita v Liberci, 460 01 Liberec (CZ)
(72) Inventor: Chvojka, Jiri, 460 01 Liberec (CZ); Lukas, David, 460 06 Liberec (CZ); Mikes, Petr, 463 31 Mnisek (CZ); Jencova, Vera, 460 06 Liberec (CZ); Horakova, Jana, 471 25 Jablonne v Podjestedi, Markvartice (CZ); Havlickova, Kristyna, 382 32 Velesin (CZ); Jirkovec, Radek, 460 05 Liberec (CZ); Nevyhosteny, Stanislav, 460 06 Liberec (CZ); Hlavata, Jana, 463 48 Vselibice (CZ); Erben, Jakub, 543 01 Vrchlabi (CZ); Klapstova, Andrea, 468 22 Koberovy (CZ)
(74) Representative: Musil, Dobroslav

(57) **Abstract**

The invention relates to a cover for acute or chronic wound which contains a functional layer consisting of a mixture of nanofibers and microfibers from a biocompatible and biodegradable copolymer of L-lactide and ε-caprolactone.

## Description

### Technical field

The present invention relates to a cover for acute or chronic wound.

### Background art

Currently known wound covers are formed by a knitted or woven textile from inert materials or contain a knitted, woven or nonwoven textile from inert materials as their surface layer intended to contact the wound. Their main disadvantage that directly arises from the structure and fiber nature of these materials is that they are susceptible to adhesion to the wound bed and therefore their subsequent removal is not only unpleasant to the user but can even cause the wound to deepen and thus extend the overall healing time. This is made worse by the fact that the existing wound covers must be periodically exchanged.

The aim of the invention is therefore to provide a wound cover which would not suffer from the above-mentioned drawbacks and thereby promote faster healing.

### Principle of the invention

The aim of the invention is achieved by a cover for acute or chronic wound whose principle consists in that it is formed by or contains as a layer to contact the wound a functional layer consisting of a mixture of nanofibers and microfibers from a biocompatible and biodegradable copolymer of L-lactide and ε-caprolactone. The cover or its functional layer, due to its chemical and morphological properties, integrates directly in the wound, and since it has a similar structure as the natural intracellular mass, it facilitates the healing process and regeneration of the damaged skin. In doing so, it gradually degrades and does not need to be removed from the wound or replaced; however, it is possible to add additional covers to the wound, or to use more covers at the same time, easily and without the user's discomfort.

The ratio of the monomer units of L-lactide and ε-caprolactone in the copolymer of the nanofibers and microfibers of the functional layer is in the range of 50:50 to 80:20, preferably 70:30.

The weight ratio of nanofibers and microfibers is 5:95 to 60:40, preferably 15:85.

The basis weight of the functional layer ranges from 5 to 30 g/m² according to the intended use, and at least one active substance, such as a growth factor and/or an antimicrobial active agent, may be incorporated in the material of the nanofibers and/or microfibers to further promote the wound healing, the active substance being released into the wound during the degradation of the fibers of the functional layer.

For ease of handling and mechanical protection, the functional layer is at least from one side partially overlaid by a cover layer formed by a textile or foil which is removed from the functional layer before or after the application of the wound cover.

### Description of drawings

In the accompanying drawings, Figs. 1 to 3 show SEM images of a functional layer of a cover for an acute or chronic wound according to the invention at 3,000 times magnification, Fig. 4 shows SEM image of the functional layer at 1,000 times magnification; Fig. 5a shows SEM image of the functional layer 24 hours after being seeded with fibroblasts at 3,000 times magnification, Fig. 5b shows a fluorescence microscopy image of the functional layer according to Fig. 5a; Fig. 6a shows SEM image of the functional layer 7 days after being seeded with fibroblasts at 3,000 times magnification, and Fig. 6b shows a fluorescence microscopy image of the functional layer of Fig. 6a.

### Examples of embodiment

The cover for an acute or chronic wound (e.g., injury, burn, surgical scar, skin damage after excision of the neoplasm, varicose ulcer, etc.) according to the invention is formed or contains as a layer intended to contact the wound a functional layer composed of a mixture of nanofibers and microfibers from compatible and biodegradable copolymer of L-lactide and ε-caprolactone. This functional layer is applied directly to the wound, to which it adheres and thanks to its chemical and morphological properties - it has a similar structure as the intercellular mass consisting predominantly of collagen and elastin fibers of nano- to micrometer diameter, it integrates to the wound, creating a structure to be intergrown by cells, thereby aiding to restore the skin without formation of a scar. At the same time, due to its structure and small interfibrous spaces, the functional layer closes the wound against external influences, but it allows the exchange of gases (especially access of fresh air from the outside) and the removal of metabolites from the wound, thereby maintaining a suitable microclimate at the site of the wound, which promotes and accelerates the healing process.

After the application of the cover, the material of the functional layer is gradually degraded to natural monomers, which are excreted by the user's body without any problems, so there is no need to subsequently treat the wound. At the same time, it is not necessary to replace the cover; however, in case of need, it is possible to add additional covers to the wound, easily and without the user's discomfort, or use simultaneously more covers.

The degradation of the material of the cover can be utilized, if necessary, to gradually release suitable active substances which promote the healing process, such as the growth factor/factors, and/or substances which inhibit infection, such as antimicrobial active substance/substances (antibiotics, active substance on base of silver or silver ions, etc.). This/these active substance/substances or its/their precursor/precursors is/are added to a copolymer solution or melt for spinning and during the subsequent production of nanofibers and/or microfibers are incorporated into their structure - see, e.g., CZ 300797.

The nanofibers and microfibers of the functional layer are either produced separately (nanofibers by electrostatic spinning, which uses DC electric current or by electric spinning, which uses an AC electric current, see, e.g., CZ 304137 or by centrifugal spinning or by meltblown technology; microfibers by electrostatic spinning or using meltblown technology or spunbond technology) and, for example, by depositing them on a common collector a mixture of microfibers is formed (which may be structured in sublayers consisting predominantly of one type of fiber), see, e.g., CZ 306018 or CZ 28190, or the two types of fibers are prepared together with a suitable adjustment of a device primarily intended for the production of nanofibres by electrostatic spinning , such as the device Nanospider™ produced by the company Elmarco. According to the process or setting used, the weight ratio of nanofibers and microfibers in the functional layer is preferably in the range of 5:95 to 60:40, the most advantageous ratio proven during experiments being about 85:15. In this functional layer, the microfibers provide especially mechanical support and reinforcement, and the nanofibers then fill the spaces between them and form a mechanical barrier blocking the impurities from entering from the outside.

The ratio of the monomer units of L-lactide to ε-caprolactone in the copolymer is preferably 50:50 to 80:20, the willingness of the copolymer solution to spin decreasing with the increasing proportion of ε-polycaprolactone. The most advantageous ratio of L-lactide to ε-caprolactone proven during experiments is about 70:30.

The basis weight of the functional layer then depends on the intended use, e.g., on the depth and/or type of the wound, and is 5 to 30 g/m², but, if necessary, it may be outside this range.

For ease of handling and mechanical protection, the functional layer is at least from one side at least partially overlaid by a cover layer consisting of a textile or foil which is removed from the functional layer before or immediately after the application of the wound cover. To provide mechanical protection, it is then possible, but not necessary, to overlay the wound cover with secondary coverings, e.g., some of the existing covering or dressing materials.

For illustrative purposes, below is a specific example of the manufacture of the cover for acute or chronic wound according to the invention.

### Example 1

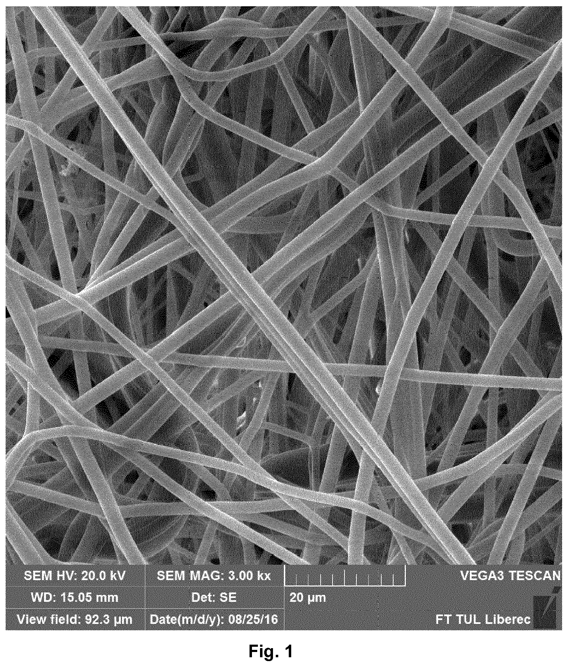

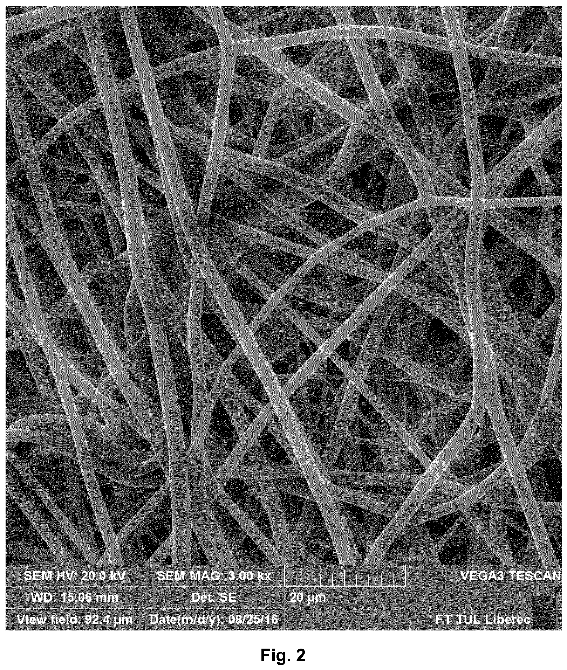

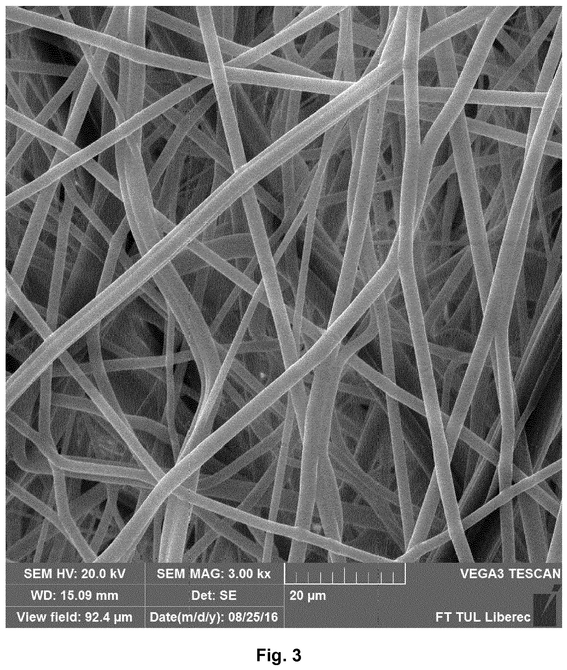

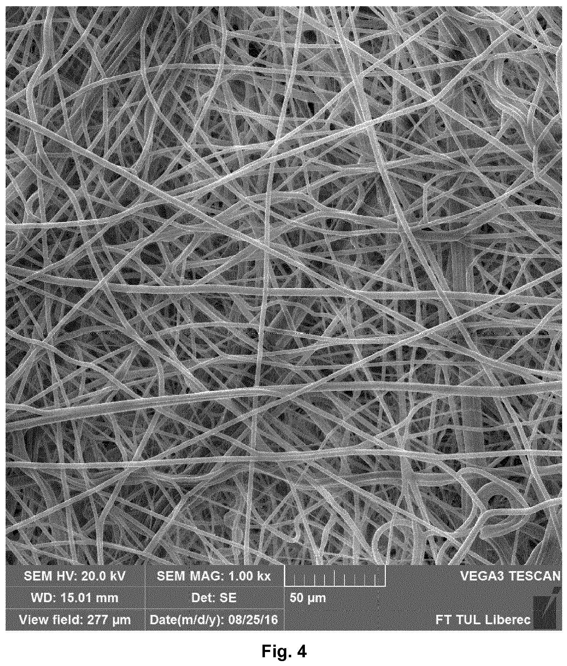

By dissolving the L-lactide and ε-caprolactone copolymer with a ratio of of L-lactide monomer units to ε-caprolactone monomer units 70:30 (Purabsorb® PLC 7015, manufactured by Corbion Purac) in a solvent system composed of chloroform and dimethylformamide in a ratio 7:3, a solution for spinning with a copolymer concentration of 10 % by weight was obtained. This solution was spun on the device Nanospider™ NS LAB produced by the company Elmarco, which was equipped with a spinning electrode formed by a cord (CZ 300345) and a collecting electrode formed by a cord, whereby the distance between the spinning electrode and the collecting electrode was 190 mm and the voltage supplied to the spinning electrode was +35 kV and to the collecting electrode - 10 kV. The resulting mixture of nanofibers and microfibers was temporarily deposited on an base nonwoven textile guided in the space between the spinning electrode and the collecting electrode. The textile was withdrawn at a velocity of 18 mm/min and a layer usable separately as a wound cover or in combination with another layer of suitable material as a functional layer of this cover was formed on it, having a basis weight of 15,8±3,1 g/m² and having a weight ratio of nanofibers to microfibers of about 15:85. Figs. 1 to 4 show SEM images of the layer at 3,000 or 1,000 times magnification.

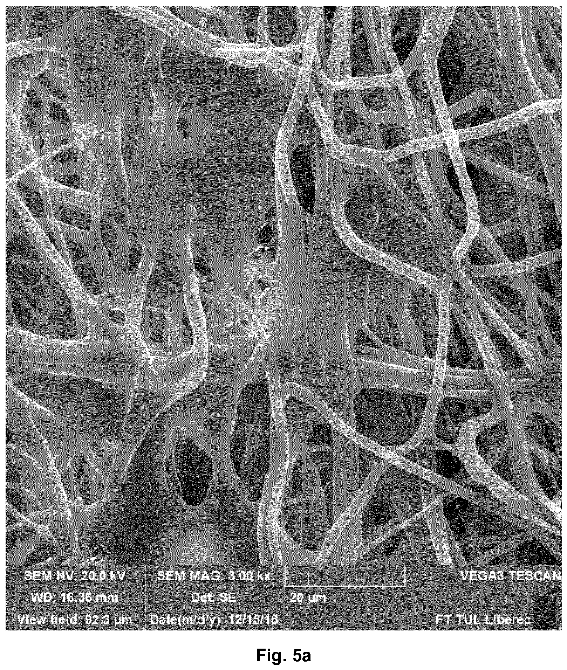

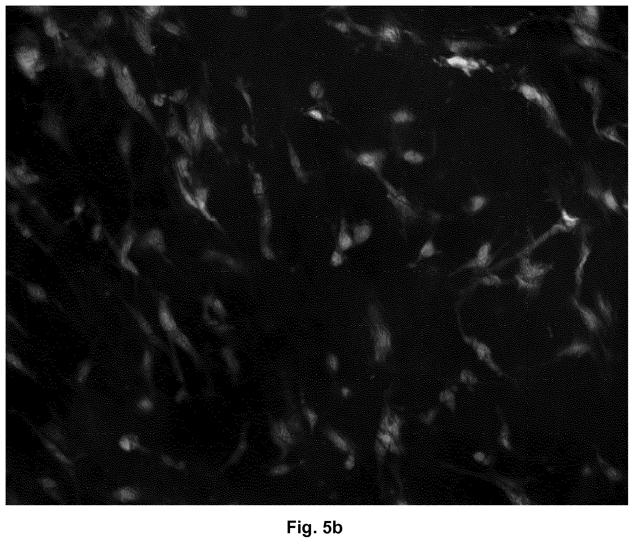

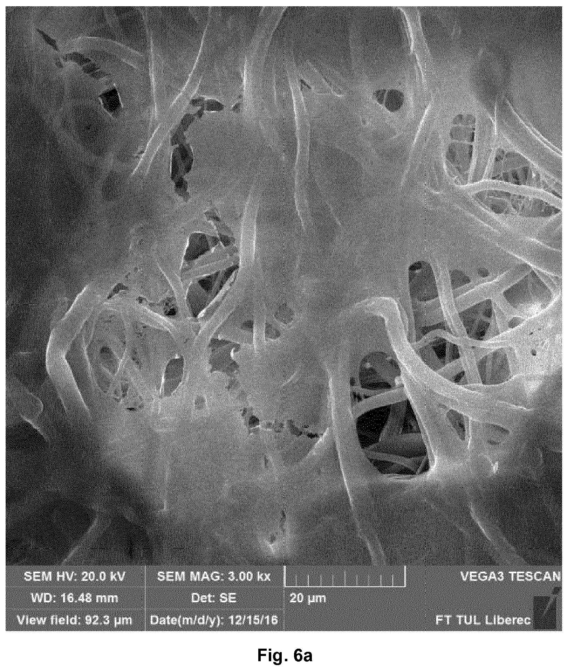

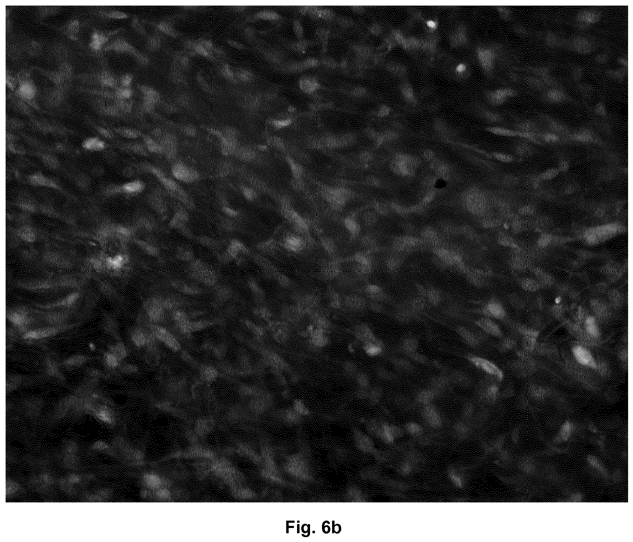

The functional layer was subsequently seeded with fibroblasts, whereby Fig. 5a shows SEM image of the layer 24 hours after being seeded with fibroblasts at 3,000 times magnification, in which the individual adhering fibroblasts can be seen, and Fig. 5b is a fluorescent microscopy image of the functional layer. Fibroblasts retained viability and colonized the functional layer - see Fig. 6a which is SEM image of the functional layer 7 days after being seeded with fibroblasts at 3,000 times magnification, and in which an almost continuous (confluent) layer of fibroblasts is already visible, and Fig. 6b which is an image of the functional layer according to Fig. 6a from a fluorescent microscope.

## Claims

1. A cover for acute or chronic wound, **characterized in that** it contains a functional layer consisting of a mixture of nanofibers and microfibers from a biocompatible and biodegradable copolymer of L-lactide and ε-caprolactone.

2. The wound cover according to claim 1, **characterized in that** the functional layer is at least from one side at least partially overlaid by a cover layer consisting of a textile or foil.

3. The wound cover according to claim 1, **characterized in that** the ratio of monomer units of L-lactide and ε-caprolactone in the copolymer of nanofibers and microfibers of the functional layer is from 50:50 to 80:20.

4. The wound cover according to claim 1 or 3, **characterized in that** the ratio of monomer units of L-lactide and ε-caprolactone in the copolymer of nanofibers and microfibers of the functional layer is 70:30.

5. The wound cover according to any of the preceding claims, **characterized in that** the weight ratio of nanofibers and microfibers in the functional layer ranges from 5:95 to 60:40.

6. The wound cover according to claim 1 or 5, **characterized in that** the weight ratio of nanofibers and microfibers in the functional layer is 15:85.

7. The wound cover according to any of the preceding claims, **characterized in that** the basis weight of the functional layer is in the range of 5 to 30 g/m².

8. The wound cover according to any of the preceding claims, **characterized in that** at least one active agent is incorporated in the nanofibers and/or microfibers of the functional layer.

9. The wound cover according to claim 8, **characterized in that the** active agent is a growth factor and/or an antimicrobial active agent
